Europäisches Patentamt

European Patent Office

Office européen des brevets

(11) Veröffentlichungsnummer: **0 063 353**
A2

## (12) EUROPÄISCHE PATENTANMELDUNG

(21) Anmeldenummer: **82103125.9**

(22) Anmeldetag: **13.04.82**

(51) Int. Cl.³: **C 07 H 21/02**
C 12 P 19/34, A 61 K 39/00
//A61K39/395

(30) Priorität: **16.04.81 DE 3115559**

(43) Veröffentlichungstag der Anmeldung:
**27.10.82 Patentblatt 82/43**

(84) Benannte Vertragsstaaten:
**AT BE CH DE FR GB IT LI LU NL SE**

(71) Anmelder: **Kommanditgesellschaft Schwarzhaupt**
**Sachsenring 37-47**
**D-5000 Köln 1(DE)**

(72) Erfinder: **Lamprecht, Walther, Prof. Dr.**
**Tiefe Trift 9**
**D-3004 Isernhagen 2(DE)**

(74) Vertreter: **Werner, Hans-Karsten, Dr. et al,**
**Deichmannhaus am Hauptbahnhof**
**D-5000 Köln 1(DE)**

(54) Hochgereinigte informatorische Ribonukleinsäure (i-RNS), Verfahren zur Herstellung und Verwendung derselben.

(57) Hochgereinigte i-RNS (informatorische Ribonukleinsäure) wird im zellfreien System synthetisiert und über einen Dichtegradienten gereinigt. Sie kann parenteral, insbesondere intravenös beim Menschen und Tier direkt oder zusammen mit anderen verträglichen Stoffen appliziert werden zwecks direkter Therapie, aktiver Immunisierung und Gewinnung von Antiseren und Antikörpern in vivo und in vitro.

Croydon Printing Company Ltd.

EP 0 063 353 A2

Hochgereinigte informatorische Ribonukleinsäure (i-RNS),

Verfahren zur Herstellung und Verwendung derselben
-----------------------------------------------------------

Gegenstand der vorliegenden Erfindung ist gegen Antigene spezifische i-RNS ("informatorische", früher auch"immune" Ribonukleinsäure benannt), die im zellfreien System gewonnen wird und so hochgereinigt ist, daß sie bei parenteraler, vorzugsweise intravenöser Applikation, beim Menschen und Tier verträglich ist. Weiterhin betrifft die Erfindung Verfahren zur Herstellung derartig hochgereinigter i-RNS sowie ihre Verwendung.

i-RNS wird normalerweise von verschiedenen immunkompetenten Zellen des Körpers nach dem Kontakt mit einem Antigen gebildet. Sie enthält und überträgt die Information für die Synthese von Antikörpern, Regulatorprotein und zellgebundene Immunität. i-RNS ist also im peripheren Blut und in lymphatischen Organen immer dann zu finden, wenn der Makroorganismus sich mit einem Antigen auseinandersetzt. Mit jeder Bluttransfusion wird eine Vielzahl von verschiedenen i-RNS-Sorten übertragen, ohne daß die mit den jeweils synthetisierten Antikörpern reagierenden Antigene bekannt sind. Die Konzentration der einzelnen i-RNS-Sorten ist jedoch zu klein, um nachweisbar zu sein.

Voraussetzung für die Gewinnung einer bestimmten i-RNS ist deshalb die experimentelle Trennung der Antigenerkennung von der Antikörpersynthese. Dies gelingt am besten in zellfreien biosynthetischen Systemen, die entweder nur das gewählte Antigen erkennen können, oder die zum anderen nur Antikörper synthetisieren können.

Aus den Arbeiten von Jachertz und anderen ist bekannt, daß das erste faßbare Produkt einer Antigenerkennung im zellfreien System die i-RNS ist. Vergl. Jachertz "Zeitschrift für medizinische Mikrobiologie und Immunologie" 154 (1968), S. 245, "Annals of the New York Academy of Science" 207 (1973), S. 122, "Zeitschrift für Immunitätsforschung und experimentelle Therapie" 144 (1972), S. 260, sowie "Journal of Immuno-Genetics" 1 (1974), S. 355-362. Die von Jachertz beschriebenen Präparationen der i-RNS sind noch stark verunreinigt gewesen und waren daher für die Applikation beim Menschen ungeeignet. Eine Verbesserung der Reinigung veröffentlichte Jachertz in "Molecular and cellular Biochemistry" 24 (1979) 93. Aber auch hier wie in den anderen Arbeiten war der experimentelle Teil so unvollständig, daß die Nacharbeitung mehrfach erfolglos blieb und daher die Ergebnisse insgesamt angezweifelt wurden. Dies führte dazu, daß weder die Ergebnisse noch die Interpretation dieser Ergebnisse von der Fachwelt akzeptiert wurden.

Es wurde auch versucht, die i-RNS in vivo herzustellen, um sie dann anschließend aus der Milz und den Lymphknoten eines Tieres zu gewinnen; vergl. Liu Shi-Shan et al. "The Lancet", January 23, 1982, S. 197. Abgesehen von der aufwendigen Herstellung (es wurden Pferde verwendet, die jeweils geschlachtet werden mußten) gelang auch hier keine vollständige Reinigung.

Die Erfindung hat sich die Aufgabe gestellt, hochgereinigte, gegen Antigene spezifische, bei Applikation, insbesondere Injektion beim Menschen verträgliche i-RNS im zellfreien System zu gewinnen. Dies ist überraschenderweise auch gelungen, wobei die so gewonnene i-RNS bei parenteraler, besonders intravenöser Applikation beim Menschen und Tier verträglich ist, wobei insbesondere an Warmblüter und Säugetiere gedacht wird.

Das erfindungsgemäße Verfahren zur Herstellung von im zellfreien System gewonnener, hochgereinigter, gegen Antigene
spezifischer, bei parenteraler, besonders intravenöser
Applikation beim Menschen verträglicher i-RNS durch Umsetzung von Antigen, DNS, (einschließlich i-DNS), Nukleotiden
und einem aus Leukozyten, Lymphozyten, sonstigen Zellen oder
Zellbestandteilen gewonnenen Enzymsystem, Extraktion mit
einer wässrigen Lösung von Phenol und Natriumdodecylsulfat
und weiterer Reinigung ist dadurch gekennzeichnet, daß man
zur Reinigung über einen Dichtegradienten zentrifugiert,
und zwar vorzugsweise über einen Dichtegradienten von
Caesiumchlorid, Saccharose und Trispuffer bei etwa 130.000 g.
Diese Zentrifugation dauert etwa 4 Stunden. Vorzugsweise
schließt sich eine weitere Zentrifugation über Caesiumsulfat
an. Besonders vorteilhaft ist es, wenn man ein Enzymsystem
verwendet, das aus Leukozyten oder Lymphozyten eines Milzhomogenisats durch Zentrifugieren über einen Dichtegradienten
gewonnen wurde. Hierfür hat sich insbesondere ein Dichtegradient von Saccharose im Trispuffer von 30 bis 60 %
Saccharose bei etwa 100.000 g bewährt. Vorteilhaft ist die
vorherige Stimulation des Immunsystems des Tieres, dessen
Milzhomogenisat zur Gewinnung des zellfreien Systems verwendet wird. Diese Stimulation kann üblicherweise mit
Freundschem Adjuvans, BCG, aber auch besonders mit Pind-Avi
(strahleninaktivierte Hühner-Pockenviren) erfolgen.

Ein weiteres Verfahren zur schonenden Reinigung besteht in
der Affinitätschromatografie. Die Matrix kann z.B. aus Poly(U)-
Sepharose 4B oder Oligo-desoxythymidin bestehen.

Die erfindungsgemäße im zellfreien System gewonnene, hochgereinigte, gegen Antigene spezifische, bei parenteraler, besonders intravenöser Applikation beim Menschen und Tier verträgliche i-RNS ist tatsächlich geeignet, bei der direkten
Therapie verwendet zu werden. Sie führt tatsächlich zur
Synthese von Antikörpern, Regulatorprotein und zellgebundener Immunität. Man kann sie daher weiterhin verwenden zur aktiven Immunisierung sowie zur Gewinnung von Antiseren und Antikörpern in vivo und in vitro. Die so gewonnenen Antiseren und Antikörper wiederum können auch zur gezielten passiven Immunisierung verwendet werden. Sie können außerdem für analytische Zwecke verwendet werden, da sie gegen die jeweiligen Antigene hochspezifisch sind. Die erfindungsgemäße i-RNS ist somit eine Schlüsselsubstanz, mit der es außerordentlich einfach und elegant und dabei ohne Nachwirkungen möglich ist, die verschiedensten Probleme zu lösen oder besser zu lösen. Selbstverständlich kann man sie auch zusammen mit anderen verträglichen Stoffen applizieren, z.B. als Zusatz zu Transfusionen von Vollblut, Vollblutkonserven, Plasma, Plasmafraktionen, Plasmaexpandern etc.

Typische Antigene sind z.B. Bakterien, Viren, Viruide, Pilze, Tumore und Parasiten. Es können aber auch höhermolekulare chemische Substanzen sein, wie Steroide, Pflanzen- und Tiergifte sowie Allergene. Besonders vorteilhaft sind strahleninaktivierte Viren, da sie nicht mehr pathogen sind, jedoch ihre Antigenizität bewahrt haben.

Es wurde gefunden, daß die erfindungsgemäße i-RNS auch im zellfreien System in der Lage ist, spezifische Antikörper zu synthetisieren. Von größerer Bedeutung ist der Befund, daß die i-RNS in Zellen die Synthese eines Regulatorproteins induziert, welches die Neusynthese von zelleigener i-RNS in der Empfängerzelle induziert. Die injizierte i-RNS und die neugebildete i-RNS sind wiederum in der Lage, die Bildung von Antikörpern zu induzieren. Die i-RNS ist somit hervorragend geeignet, zwecks spezifischer Therapie parenteral, besonders intravenös appliziert zu werden und damit die körpereigenen Abwehrkräfte rasch und gezielt zu mobilisieren. Sofern der Körper das Antigen noch nicht enthält, findet eine aktive Immunisierung statt, die rascher und komplikationsloser verläuft als die übliche Impfung mit Antigen.

Da sie einige Zeit nach der Injektion der erfindungsgemäßen i-RNS auch erhebliche Mengen an Antikörpern bilden, kann einige Zeit später das Serum für die passive Immunisierung oder Gewinnung von Antikörpern verwendet werden. Derartige Seren und Antikörper eignen sich selbstverständlich auch hervorragend für die spezifische Diagnostik und die Erkennung von Antigenen.

Die erfindungsgemäße Herstellung der i-RNS erfolgt ohne irgendwelche Manipulationen des genetischen Materials im Sinne einer Rekombination. Trotz der Synthese in zellfreien Systemen ist die erfindungsgemäße i-RNS als normaler Bestandteil des peripheren Blutes anzusehen. Kriterien der Unbedenklichkeit einer Anwendung von i-RNS beim Menschen können sich daher an den Kriterien und Erfahrungen der Bluttransfusionen orientieren. Die bisher am Menschen durchgeführten Injektionen von i-RNS haben auch in keinem Falle die geringsten Nebenwirkungen gezeigt. Die gewünschte biologische Wirkung wurde hingegen in nahezu allen Fällen registriert. Hieraus kann der Schluß gezogen werden, daß die im zellfreien System aus immunkompetenten Zellen nach der Stimulation mit Antigen gebildete i-RNS tatsächlich die gesamte notwendige Information für die Synthese von Antikörpern und für die Ausprägung zellgebundener Immunität aufweist. Weiterhin ist die i-RNS frei von Antigenen und Antigen-Bruchstücken sowie anderweitigen Verunreinigungen, wie Proteinen, DNS- und RNSdifferenten Informationsgehalten. Dies kann beispielsweise durch Agarose-Gel-Elektrophorese oder Dünnschichtchromatographie nachgewiesen werden.

Zur Durchführung der direkten Therapie reicht es im allgemeinen aus, die hochgereinigte erfindungsgemäße i-RNS einmal zu injizieren. Nur in einigen Fällen erschien es angezeigt, nach etwa 10 Tagen die Injektion zu wiederholen.

In den nachfolgenden Beispielen ist die Erfindung näher erläutert:

- 6 -

Beispiel 1

a) Herstellung des Enzymsystems

Für die Herstellung des geeigneten Enzymsystems können Leukozyten und Lymphozyten verwendet werden. Besonders einfach ist die Herstellung aus Milz. Hierzu werden 1 bis 2 g frische, tiefgefrorene Milz (z.B. Schweinemilz) mit Trockeneis und flüssigem Stickstoff im Mörser zu Pulver zerstoßen. Das Pulver wird zu 1,5 ml Tris-Puffer A in ein Zentrifugenglas gegeben und die Suspension bei -70°C eingefroren. Dieser Vorgang wird ca. 10 x wiederholt. Danach werden die aufgeschlossenen Zellen 30 Minuten in einer Beckman Ultrazentrifuge im Rotor SW 50.1 bei 30.000 g abzentrifugiert. Nach der Zentrifugation wird 1 bis 1,5 ml des roten klaren Überstandes abgenommen und auf einen diskontinuierlichen Saccharose-Gradienten gebracht. Dieser Gradient besteht aus 1 ml 60 %iger Saccharose im Tris-Puffer A, über der sich 2,75 ml 30 %ige Saccharose-Lösung im Tris-Puffer A befinden. In einer Beckman-Ultrazentrifuge, Rotor SW 50.1, wird bei 100.000 g, 0°C zentrifugiert. Nach 4 Stunden erhält man die gewünschte Fraktion als rötlich klare Schicht oberhalb der 30 %igen Saccharose. Die Schicht wird mit einer vorgekühlten Pipette abgenommen und mit kaltem Tris-Puffer A im Verhältnis 1:1 verdünnt. Die Lösung kann im flüssigen Stickstoff aufbewahrt werden.

- 7 -

b) DNS (Desoxyribonukleinsäure) einschließlich i-DNS
   (informatorischer Desoxyribonukleinsäure)

1 bis 2 g tiefgefrorene, in Stickstoff aufbewahrte
Milz werden langsam in 5 ml Tris-Puffer A aufgetaut
und im Homogenisator durch ca. 30 Kolbenbewegungen
homogenisiert. Die Suspension wird tropfenweise in
einen Erlenmeyerkolben mit 20 ml einer Emulsion von
2%iger wässriger Natriumdodezylsulfat-Lösung mit
frisch destilliertem Phenol (Verhältnis 1:1) gegeben,
dabei wird der Kolben stark geschwenkt, um eine homogene Verteilung zu erhalten. Bei zu viskoser Mischung
kann 3 %ige Kochsalzlösung zugegeben werden. Nach
24 Stunden wird die milchig-trübe Emulsion in einer
Zentrifuge 15 Minuten bei 2000 g zentrifugiert, dabei
trennen sich zwei Phasen. Die wässrige obere Phase
wird mit dem gleichen Volumen (+ 10 %) wasserfreiem
Ethanol versetzt, so daß die DNS ausfällt. Die DNS
wird mit einer ausgeglühten Platinöse in 10 ml steriler, 0,85 %iger Natriumchlorid-Lösung überführt.
5 ml 1 %ige Natriumdodezylsulfat-Lösung, 5 ml 0,05 mol/l
Tris-A und 0,05 mol/l EDTA-Puffer pH 7,6 und 2 mg
Proteinase K werden zugefügt und gemischt. Das Auflösen der DNS dauert etwa einen Tag, danach kann sie
wieder mit Alkohol gefällt werden. Das Lösen und erneute Ausfällen wird 3 bis 7 mal wiederholt, bis das
Verhältnis von $E_{260}$ zu $E_{280}$ einen Wert zwischen 1,9
und 2 erreicht hat und der hyperchrome Effekt mindestens 20 % Extinktionszunahme bei 260 nm beträgt.
Eine Absorption von DNS bei 260 nm von 1 o.D. entspricht etwa einer Konzentration von 40 µg/ml. Mit
Hilfe dieser Relation wird die DNS-Lösung so verdünnt, daß man eine Konzentration von 1 µg/ml erhält. Diese Gebrauchslösung wird in Portionen zu
0,5 ml eingefroren und bei -20°C aufbewahrt.

- 8 -

c) Synthese der i-RNS (informatorischer Ribonukleinsäure)

Im Eisbad werden das gereinigte Enzymsystem und das Antigen in Tris-Puffer A bei pH 8,0 gegeben und mit einer Mischung der Nukleotide ATP, GTP, CTP und UTP gegeben. Zur Kontrolle wird ein kleinerer Ansatz im gleichen Mengenverhältnis erstellt, dem jedoch Tritium-markiertes UTP zugesetzt ist. Es wird stets mit gekühlten Pipetten pipettiert. Die optimalen Konzentrationen der einzelnen Komponenten werden durch mehrere Versuche ermittelt. Jede der Proben wird bei 37°C im Wasserbad inkubiert und bereits nach 3 bis 6 Minuten mit einer wässrigen Lösung von 1 % Natriumdodezylsulfat und 1 % wässrigem Phenol versetzt. Auch die optimale Inkubationszeit hängt etwas von den Konzentrationen und dem jeweiligen Antigen ab und kann durch einige Handversuche vorab ermittelt werden. Es wird im Eisbad gekühlt und bei 2.000 g abzentrifugiert. Der Überstand enthält die synthetisierte rohe i-RNS.

d) Reinigung der i-RNS

Aus dem Überstand wird die i-RNS über einen diskontinuierlichen Caesiumchlorid/Saccharose-Gradienten isoliert. Dazu wird im Zentrifugenröhrchen aus Celluloseacetat je 1 ml Caesiumchlorid-Lösung der Dichte g = 1,9 g/ml mit 0,75 ml einer 30%igen Saccharoselösung in Tris-Puffer A pH 8,0 überschichtet. Je 2 ml der Proben werden auf die Gradienten gegeben und die Röhrchen mit sterilem Paraffin verschlossen. Nach dem Zentrifugenlauf bei 18 °C, 130.000 g, 4 Stunden im Beckman Rotor SW 50.1 wird die Caesiumchlorid-Schicht abgenommen. Hierzu werden die Zentrifugenröhrchen mit einer ausgeglühten Nadel am Boden angestochen und die gesamte Caesiumchlorid-Lösung bis zur Saccharose-Schicht in Röhrchen überführt. Die so erhaltene Lösung wird mit sterilem Wasser 1:2 verdünnt und kann eingefroren bei -20°C aufgehoben werden. Die weitere Reinigung erfolgt, indem man über einen Caesiumsulfat-Gradienten sedimentiert. Dazu werden 4,8 ml Caesiumsulfat-Lösung der Dichte 1,5 g/ml in Zentrifugenröhrchen aus Celluloseacetat gefüllt (Rotor Beckman SW 50.1). Darüber schichtet man 0,3 ml der Probe und zentrifugiert mindestens 4 Stunden (vorzugsweise 12 bis 19 Stunden) bei 18° C und 130.000 g. Nach dem Lauf werden die Röhrchen am Boden durchstochen und 13 - 14 Fraktionen zu je 9 Tropfen (= 0,4 ml) in Fraktionen geschnitten. 0,1 ml jeder Fraktion werden zur Bestimmung der Radioaktivität verwendet, die restlichen 0,3 ml werden mit dem gleichen Volumen Wasser verdünnt und bei -20°C aufbewahrt. Nachdem festgestellt wird, in welchen Fraktionen die höchste Radioaktivität enthalten ist, werden auch die entsprechenden Fraktionen des Hauptansatzes ausgewählt. Sie enthalten die hochgereinigte i-RNS.

Die Fraktionen können gewünschtenfalls ohne weitere Reinigungsschritte unmittelbar injiziert werden.

Es ist möglich, die i-RNS elektronenoptisch nachzuweisen, wobei Moleküllängen zwischen 1.500 und 2.000 nm beobachtet wurden. Der Reinheitsgrad läßt sich beispielsweise durch Agarose-Gel-Elektrophorese nachweisen.

Ein weiterer Nachweis der i-RNS besteht darin, daß man im zellfreien System Antikörper synthetisiert. Hierzu werden eine Probe der i-RNS, hochgereinigter Ribosomen, des

- 10 -

oben erwähnten Enzymsystems und Leuzin und Valin
in Tris-Puffer A pH 8,0 zusammengegeben. Die Proben werden
45 Sekunden bei 7°C inkubiert und danach die Proteinsynthese durch Einfrieren bei -20°C gestoppt. Die Antikörper können durch Affinitätschromatographie an
Immunadsorptionssäulen aufgetrennt und gereinigt
werden und analytisch nachgewiesen werden.

Die höchsten Konzentrationen an Antikörpern werden
erzeugt bei Zusatz von nur $10^{-4}$ i-RNS. Dies zeigt, daß
ein Molekül i-RNS im zellfreien System in der Lage ist,
mindestens $10^4$ Moleküle Antikörper zu synthetisieren.

Es ist weiterhin möglich, mit einer RNS-Replikase
die Menge an i-RNS um den Faktor $10^4$ bis $10^5$ zu erhöhen. Durch mehrfache Wiederholung dieser Schritte
findet eine Klonisierung statt. Da auch die so erhaltene i-RNS noch die gleichen Eigenschaften bezüglich der Antikörpersynthese hat wie die ursprünglich
gewonnene, kann ausgeschlossen werden, daß Antikörper
noch durch verschleppte Antigenreste erzeugt wurden.

Die Analyse der i-RNS ergab, daß das Molekulargewicht
etwa $1 - 2 \times 10^6$ beträgt. Als Antigene wurden
Aujeszky-Virus,        Herpes-simplex-Virus,        Influenza-Virus, MKS-Virus, Varicella-Virus, Masernvirus, HBs-Antigen, E.coli O-Antigen, Schafserythrozyten, ADH, Tetanustoxin, sowie Systeme mit
Tumorzellen, wie $LC_2$ von Meerschweinchen, p815
von der $DBA_2$-Maus, L 1210 von der $DBA_2$-Maus sowie
der durch Poliomavirus induzierte Tumor bei Lewis-
Ratten eingesetzt. Erste Erfolge wurden weiterhin
erzielt mit dem attenuierten Masernvirus "Moraten"

- 11 -

und HBS-Antigen. Entsprechende Versuche zeigten, daß keine Kreuzreaktionen eintreten. Es entstehen somit hochspezifische i-RNS in Abhängigkeit vom jeweils eingesetzten Antigen.

Beispiel 2

Gegen die Hepatitis B wurde eine spezifische i-RNS hergestellt und an über 200 Patienten auf Verträglichkeit und Wirksamkeit geprüft. Die i-RNS-Dosis betrug $10^{-5}$ Mol/ml. Die Patienten erhielten von dieser Lösung in den meisten Fällen einmal ein bis zwei ml, in seltenen schweren Fällen wurde eine zweite Injektion durchgeführt. Die derzeit überschaubare Heilungsquote beträgt nahezu 100 %.

Beispiel 3

BALB/c-Mäusen wurden in wöchentlichen Abständen dreimal je 100 µl komplettes Freundsches Adjuvans gespritzt. Das käufliche komplette Freundsche Adjuvans wurde 1 : 1 mit physiologischer Kochsalzlösung gemischt. Nach der letzten Injektion wurde 4 Wochen gewartet. Danach wurde die Milz der Tiere entfernt und zur Gewinnung des zellfreien Systems aufgearbeitet.

Die mit diesem zellfreien System erzielten Ausbeuten an i-RNS waren um den Faktor 10 höher.

Patentansprüche

1. Im zellfreien System gewonnene, hochgereinigte,
   gegen Antigene spezifische, bei Applikation beim
   Menschen und Tier verträgliche i-RNS.

2. i-RNS gemäß Anspruch 1, dadurch gekennzeichnet, daß
   sie bei parenteraler, besonders intravenöser Applikation
   verträglich ist.

3. Verfahren zur Herstellung von im zellfreien System gewonnener, hochgereinigter, gegen Antigene spezifischer,
   bei parenteraler, besonders intravenöser Applikation
   beim Menschen und Tier verträglicher i-RNS durch Umsetzung von Antigen, DNS (einschließlich i-DNS), Nukleotiden und einem aus Leukozyten, Lymphozyten, sonstigen
   Zellen oder Zellbestandteilen gewonnenen Enzymsystem,
   Extraktion mit einer wässrigen Lösung von Phenol und
   Natriumdodecylsulfat und weiterer Reinigung, dadurch
   gekennzeichnet, daß man zur Reinigung über einen Dichtegradienten zentrifugiert.

4. Verfahren gemäß Anspruch 3, dadurch gekennzeichnet, daß
   man über einen Dichtegradienten von Caesiumchlorid/
   Saccharose und Trispuffer bei etwa 130.000 g zentrifugiert.

5. Verfahren gemäß Anspruch 4, dadurch gekennzeichnet, daß
   man anschließend die i-RNS mittels Affinitätschromatografie reinigt.

6. Verfahren gemäß Anspruch 4, dadurch gekennzeichnet, daß
   man anschließend über Caesiumsulfat zentrifugiert.

7. Verfahren nach einem der Ansprüche 3 bis 6, dadurch gekennzeichnet, daß man ein Enzymsystem verwendet, das gewonnen wird durch Reinigung eines Homogenisats aus Gewebe des retikulo-endothelialen Systems (RES), vorzugsweise Milz durch Zentrifugieren über einen Dichtegradienten.

8. Verfahren gemäß Anspruch 7, dadurch gekennzeichnet, daß das Immunsystem der Tiere, die zur Gewinnung des Homogenisats aus Gewebe des retikulo-endothelialen Systems verwendet werden, vorher stimuliert wird, vorzugsweise mit BCG, Freunds hem Adjuvans und Pind-Avi.

9. Verfahren gemäß Anspruch 7, dadurch gekennzeichnet, daß man über einen Dichtegradienten von Saccharose im Trispuffer bei etwa 100.000 g zentrifugiert.

10. Verwendung von im zellfreien System gewonnener, hochgereinigter, gegen Antigene spezifischer, bei parenteraler, vorzugsweise intravenöser Applikation beim Menschen und Tier verträglicher i-RNS zur direkten Therapie, zur aktiven Immunisierung oder Gewinnung von Antiseren und Antikörpern in vivo und in vitro.

11. Verwendung gemäß Anspruch 10, dadurch gekennzeichnet, daß die Applikation zusammen mit anderen verträglichen Stoffen erfolgt.